# EUROPÄISCHE PATENTSCHRIFT

(11) **EP 2 049 016 B1**
(45) Veröffentlichungstag und Bekanntmachung des Hinweises auf die Patenterteilung: **18.07.2012**
(21) Anmeldenummer: 07786659.8
(22) Anmeldetag: 10.08.2007
(51) Int. Cl.: A61B 6/00, A61B 6/03, F16C 32/06, G01N 23/04

(54) **VORRICHTUNG MIT EINEM DIREKT ANGETRIEBENEN ROTATIONSKÖRPER UND EINEM AEROSTATISCHEN LAGER**
DEVICE HAVING A DIRECTLY DRIVEN ROTATING BODY AND AN AEROSTATIC BEARING
DISPOSITIF POURVU D'UN CORPS DE ROTATION A ENTRAINEMENT DIRECT ET D'UN PALIER AEROSTATIQUE

(30) Priorität: 10.08.2006 DE 102006037543
(43) Veröffentlichungstag der Anmeldung: 22.04.2009
(73) Patentinhaber: AeroLas GmbH Aerostatische Lager-Lasertechnik, 82008 Unterhaching (DE)
(72) Erfinder: MUTH, Michael, 81737 München (DE)
(74) Vertreter: Schlimme, Wolfram
(86) Internationale Anmeldenummer: PCT/EP2007/007098
(87) Internationale Veröffentlichungsnummer: WO 2008/017498

(56) Entgegenhaltungen:
- EP-A- 0 215 523
- WO-A-02/089671
- WO-A-2005/102171
- WO-A-2006/089741
- DE-A1- 3 815 029
- DE-A1- 19 745 216
- DE-C1- 4 436 156
- US-A- 3 351 394
- US-A- 5 608 771
- US-A1- 2004 017 895
- US-A1- 2004 062 356
- US-A1- 2005 116 558

## Beschreibung

Die Erfindung betrifft eine Vorrichtung mit einem direkt angetriebenen Rotationskörper gemäß dem Oberbegriff des Patentanspruchs 1.

Eine gattungsgemäße Vorrichtung und ist aus der WO 02/089671 A2 bekannt. Diese Druckschrift offenbart ein aerostatisches Rotorlager für die rotierende, ringförmige Gantry eines Computertomographen. Die kreisringförmige, rotierende Gantry ist an ihrem Außenumfang an vier in Umfangsrichtung voneinander beabstandeten Positionen mittels jeweils eines Fluiddrucklagers gelagert. Durch diese Vierpunktlagerung wird eine statische Überbestimmtheit des Lagers erzielt, die dadurch kompensiert wird, dass die beiden oberen Lager justierbar ausgebildet sind. Der Antrieb dieser rotierenden Gantry erfolgt durch einen externen Motor. Zur axialen Lagerung der rotierenden Gantry sind Fluidlager vorgesehen, die mit den beiden voneinander abgewandten Stirnseiten der ringförmigen Gantry zusammenwirken. Die Fluidlager, die einer der beiden Stirnseiten zugeordnet sind, sind justierbar. Der Aufbau derartiger Computertomographen ist allgemein bekannt, beispielsweise aus dieser Druckschrift, auf deren Offenbarung bezüglich des Aufbaus eines Computertomographen hier ausdrücklich Bezug genommen wird.

An der ringförmigen Gantry eines Computertomographen ist im allgemeinen eine bildgebende Einrichtung angebracht, die zusammen mit der ringförmigen Gantry um einen zu untersuchenden Bereich rotiert.

Um bei Untersuchungen mit einem Computertomographen auch bewegte Körper, wie beispielsweise ein schlagendes Herz, scharf abbilden zu können, ist es erforderlich, daß die rotierende Gantry mit einer ausreichend hohen Drehzahl und präzise geführt rotiert. Die hierfür erforderlichen hohen Drehzahlen lassen sich jedoch wegen des bauartbedingten großen Durchmessers der Gantry und der damit verbundenen hohen Umfangsgeschwindigkeit der Lagerkörper mit wälzgelagerten Anordnungen nicht mehr erzielen, weswegen bereits in der Vorrichtung gemäß der WO 02/089671 A2 aerostatische Lager eingesetzt werden.

Damit aerostatische Lager zuverlässig funktionieren, ist es erforderlich, daß der Lagerspalt zwischen den einander gegenüberstehenden Lagerflächen klein gehalten wird, also der Abstand zwischen den einander gegenüberstehenden Lagerflächen sehr gering ist.

Aufgrund des großen Durchmessers der Gantry und der an der Gantry asymmetrisch angebrachten Einrichtungen zur Bildgebung entstehen insbesondere bei hohen Drehzahlen Verformungen der ringförmigen Gantry, sodaß eine definierte Größe des Lagerspalts über den gesamten Umlauf der ringförmigen Gantry nicht gewährleistet ist. Die WO 02/089671 A2 lehrt zwar, daß mindestens einige der aerostatischen Lager justierbar sind, um so den Luftspalt genau einstellen zu können, doch lassen sich hierdurch Verformungen des Rotors nicht ausgleichen. Insbesondere, wenn die ringförmige Gantry direkt angetrieben ist, kann es wegen der zwischen dem stationären Körper und der rotierenden Gantry wirkenden Magnetkräfte des Antriebs zu wechselnden magnetischen Radialkräften führen, die eine zusätzliche bereichsweise Verformung der ringförmigen rotierenden Gantry bewirken und zudem eine dynamische Unwucht hervorrufen, die als "magnetische Unwucht" bezeichnet werden kann und die auch durch die justierbaren Lager der WO 02/089671 A2 nicht ausgeglichen werden kann.

Die US 2004/0017895 A1 offenbart einen Computertomographen, bei welchem eine rotierende Gantry mit einem linearmotorartigen Antrieb verbunden ist, dessen Rotor sich in Radialrichtung erstreckt und dessen mit der stationären Gantry verbundener Stator zwei Statorwicklungen aufweist, die den Rotor zangenartig derart umgreifen, dass die Kraftlinien zwischen Rotor und Stator achsparallel verlaufen. Durch das Vorsehen von zwei Statorwicklungen, die den Rotor zangenartig umgreifen, wird der Rotor in Axialrichtung in einer Gleichgewichtslage zwischen den beiden Statorwicklungen gehalten. Die in Axialrichtung wirkenden Kräfte zwischen dem Rotor und der ersten Statorwicklung sowie zwischen dem Rotor und der zweiten Statorwicklung heben sich dabei gegenseitig auf. Der Rotor ist in der stationären Gantry mittels eines Kugellagers gelagert.

Aufgabe der vorliegenden Erfindung ist es daher, eine gattungsgemäße Vorrichtung anzugeben, die die Auswirkungen einer "magnetischen Unwucht" auf den direkt angetriebenen Rotationskörper deutlich reduziert und die eine Rotationsgeschwindigkeit zuläßt, die höher ist als im bisher bekannten Stand der Technik.

Die die Vorrichtung betreffende Aufgabe wird durch die im Patentanspruch 1 angegebenen Merkmale gelöst.

Durch das Vorsehen des elektrischen Direktantriebs mit dessen ringförmiger Magnetanordnung und dessen ringförmiger Spulenanordnung an einer Stirnseite des Rotationskörpers wird bewirkt, daß die Magnetfeldlinien zwischen der Magnetanordnung und der Spulenanordnung und damit die zwischen diesen beiden Bestandteilen des elektrischen Direktantriebs wirkenden Anzugskräfte in Axialrichtung - und nicht wie bei herkömmlichen Computertomographen in Radialrichtung - verlaufen. Änderungen der Magnetkräfte dieses Antriebs führen somit nicht zu einer lokalen Radiusänderung des Rotationskörpers, sondern können lediglich zu einer lokalen Verformung des Rotationskörpers in Axialrichtung führen. Das hat zur Folge, daß die Umfangskontur des Rotationskörpers während dessen Umlaufs im wesentlichen konstant bleibt, also keine in Radialrichtung wirkenden Verformungen und/oder "magnetischen Unwuchten" auftreten. Folglich bilden die Radiallager jeweils einen während des Umlaufs des Rotationskörpers im wesentlichen konstanten mittleren Abstand zwischen den Lagerflächen aus, wodurch eine zuverlässige Funktionsfähigkeit der Radiallager gewährleistet ist, insbesondere wenn diese als aerostatische Lager ausgebildet sind.

Insbesondere sind auch die Radiallager durch aerostatische Lager gebildet, wodurch sich ebenfalls in den Radiallagern eine nahezu reibungsfreie Lagerung des Rotationskörpers realisieren läßt.

In einer bevorzugten Ausführungsform der vorliegenden Erfindung ist der von der ersten Stirnseite des Rotationskörpers abgewandten zweiten Stirnseite zugeordnet zumindest ein weiteres aerostatisches Axiallager vorgesehen, das jeweils zumindest einen in achsparalleler Richtung bewegbaren Lagerschuh aufweist, der mittels einer Federkraft oder einer pneumatischen oder hydraulischen Kraft gegen die zweite Stirnseite des Rotationskörpers vorgespannt ist und ein axiales Ausgleichslager bildet. Auf diese Weise wird der Rotationskörper in axialer Richtung zwischen den auf seinen beiden Stirnseiten angeordneten Lagern quasi schwimmend gelagert, wobei das oder die auf der zweiten Stirnseite vorgesehenen Lager mit bewegbarem Lagerschuh nicht nur eine Vorspannung des jeweiligen axialen Lagers erzeugen, sondern darüber hinaus eine axiale Verschiebung der jeweiligen Lagerfläche des jeweiligen der zweiten Stirnseite zugeordneten Lagerschuhs ermöglichen, sodaß auf diese Weise eine durch die "magnetische Unwucht" hervorgerufene lokale axiale Verformung des Rotationskörpers geometrisch ausgeglichen werden kann, da der Lagerschuh in Axialrichtung der lokalen zyklischen Verlagerung des Rotationskörpers in Axialrichtung folgen kann.

Vorzugsweise ist unterhalb des Rotationskörpers zumindest ein weiteres aerostatisches Radiallager vorgesehen, das zumindest einen in radialer Richtung bewegbaren Lagerschuh aufweist, der mittels einer Federkraft oder einer pneumatischen oder hydraulischen Kraft gegen den Außenumfang des Rotationskörpers drückt. Dieses radiale Ausgleichslager weist die gleichen prinzipiellen Vorteile auf wie die vorstehend beschriebenen axialen Ausgleichslager.

Vorzugsweise ist der ersten Stirnseite des Rotationsantriebs zugeordnet zumindest ein weiteres aerostatisches Axiallager vorgesehen, das zumindest einen in achsparalleler Richtung bewegbaren Lagerschuh aufweist, der mittels einer Federkraft oder einer pneumatischen oder hydraulischen Kraft gegen die ersten Stirnseite des Rotationskörpers vorgespannt ist. Dieses zusätzliche Axiallager mit bewegbarem Lagerschuh auf der ersten Lagerseite erhöht die Tragfähigkeit der der ersten Stirnseite zugeordneten Lager, ohne jedoch die durch die vorzugsweise drei grundsätzlich zur axialen Lagerung vorgesehenen aerostatischen Lager definierte statische Bestimmtheit der Lagerung zu beeinträchtigen und ohne folglich zu einer statischen Überbestimmtheit dieser Lagerung zu führen.

Besonders vorteilhaft ist es, wenn zumindest ein Teil der Axiallager jeweils zumindest einen schwenkbaren Lagerschuh aufweist, der um eine senkrecht zur Rotationsachse des Rotationskörpers verlaufende erste Achse schwenkbar ist. Ein derart schwenkbarer Lagerschuh ermöglicht es, die durch aerodynamische Kräfte, welche bei der Rotation des Rotationskörpers im Bereich des Lagers auftreten, bedingte ungleiche Belastung des Lagerschuhs zu kompensieren.

Insbesondere ist dazu der jeweilige schwenkbare Lagerschuh vorteilhafterweise auch um eine zweite Achse schwenkbar, die senkrecht zu einer von der ersten Achse und der Rotationsachse aufgespannten Ebene verläuft.

Besonders vorteilhaft ist es jedoch, wenn der schwenkbare Lagerschuh ein Kugelkopflager aufweist und so beliebig schwenkbar ausgebildet ist.

Die vorgenannte Schwenkbarkeit des Lagerschuhs um eine Achse, zwei rechtwinklig zueinander stehende Achsen oder gar um einen Kugelmittelpunkt wird vorteilhafterweise auch bei den Radiallagern realisiert.

Besonders vorteilhaft ist es, wenn die jeweiligen Lagerschuhe so ausgestaltet sind, daß in Bewegungsrichtung der sich am Lagerschuh vorbeibewegenden Gegenlagerfläche die Tragkraft des Lagers zunimmt. Hierdurch kann die sich aufgrund der aerodynamischen Effekte einstellende Schrägstellung des Lagers, die zu einer relativen Positionierung der Lagerflächen zueinander führt, bei der der Lagerspalt eine im Querschnitt keilförmige Gestalt aufweist, durch die sich über die Längserstreckung des Lagerschuhs ändernde Tragfähigkeit kompensiert werden.

Diese sich über die Längsrichtung des Lagers verändernde Tragkraft kann in vorteilhafter Weise durch eine sich in Längsrichtung des Lagers verändernde Anzahl der Gasaustrittsöffnungen derart erzielt werden, daß die Anzahl der Gasaustrittsöffnungen in der Lagerfläche des Lagerschuhs in Bewegungsrichtung der sich am Lagerschuh vorbeibewegenden Gegenlagerfläche zunimmt.

Die erfindungsgemäße Vorrichtung kann in einem Tomographen realisiert sein, sie kann aber auch in jeder anderen eine stationäre Gantry und eine rotierende Gantry aufweisenden Einrichtung realisiert sein.

Insbesondere das erfindungsgemäße um eine Achse, um zwei Achsen oder um einen Kugelmittelpunkt schwenkbare aerostatische Lager mit vorzugsweise sich über eine Längserstreckung des Lagerschuhs ändernder Tragkraft des Lagers ist nicht auf die Anwendung in einem Tomographen beschränkt. Sie kann bei jeder Lagerung eines schnell bewegten Körpers realisiert sein, so beispielsweise auch bei der Lagerung von Rotoren anderer Motoren oder Generatoren. Die Erfindung wird nachfolgend anhand eines Beispiels unter Bezugnahme auf die Zeichnung näher erläutert; in dieser zeigt:
- **Fig. 1.**: eine Draufsicht auf eine erfindungsgemäße Vorrichtung in Axialrichtung;
- **Fig. 2.**: eine geschnittene Darstellung der Vorrichtung aus Fig. 1 entlang der strichpunktierten Linie II-II;
- **Fig. 3.**: eine vergrößerte Darstsellung der Einzelheit III in Fig. 2 und
- **Fig. 4.**: einen erfindungsgemäßen Lagerschuh mit sich über die Längsrichtung des Lagerschuhs verändernder Tragfähigkeit in perspektivischer Darstellung.

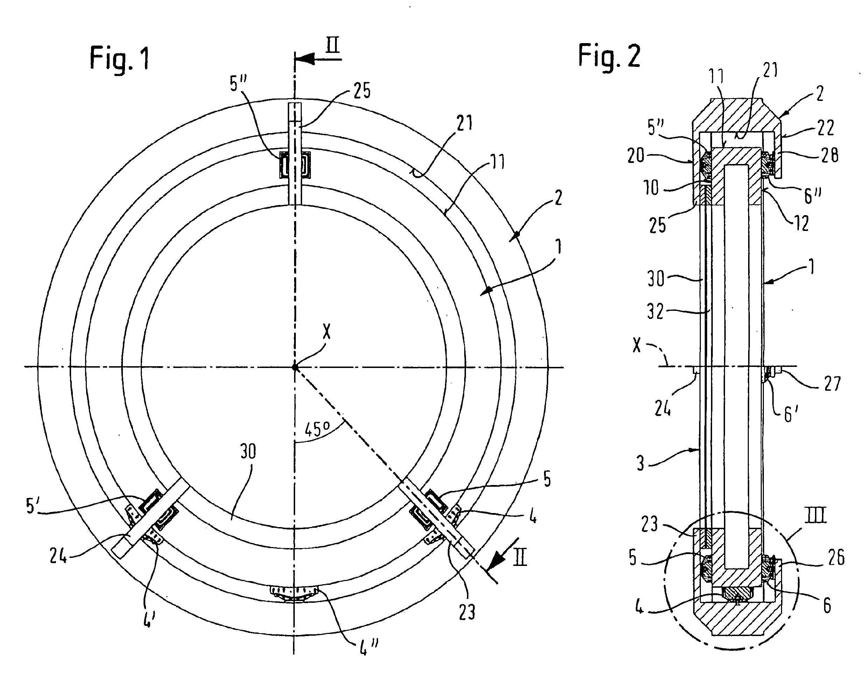

In den Fig. 1 und 2 ist eine erfindungsgemäße Vorrichtung dargestellt, wie sie beispielsweise in einem Tomographen realisiert ist. Ein ringförmiger Rotationskörper 1 ist im Inneren eines stationären Körpers 2 angeordnet. Der äußere, stationäre Körper 2 kann dabei die stationäre Gantry eines Computertomographen sein und der innere Rotationskörper 1 kann die rotierende Gantry eines Computertomographen sein, an welcher die bildgebende Einrichtung (nicht gezeigt) des Computertomographen angebracht ist.

An einer ersten Stirnseite 20 des stationären Körpers 2 sind drei radial einwärts gerichtete radiale Stege 23, 24, 25 vorgesehen, die an ihrem radial inneren Ende eine ringförmige elektrische Spulenanordnung 30 des axialen elektrischen Direktantriebs 3 tragen.

Der Spulenanordnung 30 in Axialrichtung unmittelbar gegenüber gelegen ist eine ringförmige Magnetanordnung 32 des elektrischen Direktantriebs 3 an einer ersten Stirnseite 10 des Rotationskörpers 1 vorgesehen. Sowohl die ringförmige Magnetanordnung 32, als auch die ebenfalls ringförmig ausgebildete elektrische Spulenanordnung 30 sind koaxial zur Drehachse X des Rotationskörpers 1 angeordnet.

Die erfindungsgemäße Vorrichtung ist vertikal ausgerichtet, sodaß die Drehachse X horizontal verläuft. Ein erstes Radiallager 4 und ein zweites Radiallager 4' sind jeweils um 45° in Umfangsrichtung zur einen Seite beziehungsweise zur anderen Seite des tiefsten Punktes des Innenumfangs 21 des stationären Körpers 2 versetzt an diesem angeordnet und bilden Stützlager, die den Rotationskörper 1 abstützen. Alternativ können die Radiallager 4, 4' auch um jeweils 60° oder einen anderen Winkel kleiner als 90° bezüglich des tiefsten Punktes versetzt sein. Das erste Radiallager 4 ist dabei im Bereich des ersten radialen Stegs 23 gelegen und das zweite Radiallager 4' ist im Bereich des zweiten radialen Stegs 24 gelegen. Der dritte radiale Steg 25 ist am höchsten Punkt des Innenumfangs 21 des stationären Körpers 2 angebracht.

Am tiefsten Punkt des Innenumfangs 21 des stationären Körpers 2 ist ein drittes Radiallager 4" angebracht, das den Rotationskörper 1 in Vertikalrichtung auf dem stationären Körper 2 zusätzlich abstützt und das ein radiales Ausgleichslager bildet.

Der jeweilige Aufbau der Radiallager 4, 4', 4" wird weiter unten im Detail beschrieben.

An jedem der drei radialen Stege 23, 24, 25 des stationären Körpers 2 ist auf der zum Rotationskörper 1 gewandten Innenseite ein Axiallager 5, 5', 5" vorgesehen, das jeweils eine zur ersten Stirnseite 10 des Rotationskörpers 1 gewandte Lagerfläche aufweist.

Der Aufbau der Axiallager 5, 5', 5" wird weiter unten noch beschrieben.

Auf der von der ersten Stirnseite 20 des stationären Körpers 2 abgewandt gelegenen zweiten Stirnseite 22 sind ebenfalls drei radial einwärts gerichtete radiale Stege 26, 27, 28 vorgesehen, die an jeweils demselben Umfangsort ausgebildet sind wie die ersten radialen Stege 23, 24, 25 und von denen jeweils ein Steg 26, 27, 28 mit einem zugeordneten der ersten radialen Stege 23, 24, 25, in Axialrichtung gesehen fluchtet. Die zweiten radialen Stege 26, 27, 28 sind in Radialrichtung gesehen kürzer als die ersten radialen Stege 23, 24, 25.

An jedem der zweiten radialen Stege 26, 27, 28 ist, der zweiten Stirnseite 12 des Rotationskörpers 1 gegenüber gelegen, jeweils ein axiales Ausgleichslager 6, 6', 6" angeordnet, dessen jeweilige Lagerfläche mit einer an der zweiten Stirnseite 12 des Rotationskörpers 1 vorgesehenen Gegenlagerfläche zusammenwirkt.

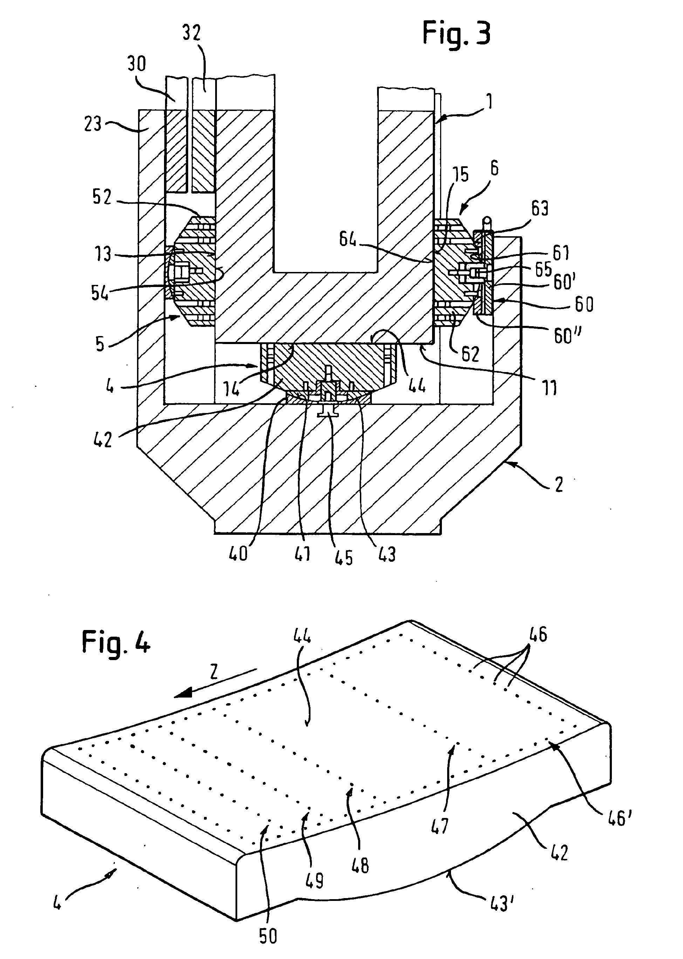

Nachfolgend wird unter Bezugnahme auf Fig. 3 der Aufbau eines jeweiligen radialen Stützlagers 4, 4' beschrieben.

Das jeweilige Radiallager 4, 4' umfaßt eine Lagerbasis 40 und einen Lagerschuh 42. Die Lagerbasis 40 ist am stationären Körper 2 befestigt. Auf der vom stationären Körper 2 abgewandten Seite ist die Lagerbasis 40 mit einer kugelig-konkaven Fläche 41 versehen, gegen welche eine entsprechend kugelig-konvex gekrümmte Fläche 43 anliegt, die an der zur Lagerbasis 40 gewandten Rückseite des Lagerschuhs 42 ausgebildet ist. Auf der von der konvexen Fläche 43 abgewandten Seite des Lagerschuhs 42 ist die Lagerfläche 44 ausgebildet. Die Lagerbasis 40 und der Lagerschuh 42 sind über einen Zuganker 45 derart miteinander verbunden, daß eine allseitige Schwenkbewegung des Lagerschuhs 42 um den gemeinsamen Kugelmittelpunkt der konkaven Fläche 41 und der konvexen Fläche 43 um einen vorgegebenen Winkel ermöglicht ist. Die beiden gekrümmten Flächen 41 und 43 bilden ein Kugelkopflager für den Lagerschuh 42.

Die Lagerfläche 44 weist eine Krümmung auf, die an die Krümmung des Außenumfangs 11 des Rotationskörpers 1 so angepaßt ist, daß im Idealzustand ein gleichmäßig dicker Lagerspalt zwischen der Lagerfläche 44 und der auf dem Außenumfang 11 des Rotationskörpers 1 ausgebildeten Gegenlagerfläche 14 besteht.

Die allseitige Schwenkbarkeit des Lagerschuhs 42 bezüglich des stationären Körpers 2 ermöglicht es, auch dann, wenn die Rotationsachse X des Rotationskörpers 1 bezüglich der zentralen Achse des kreisförmigen Innenumfangs 21 des stationären Körpers 2 geneigt ist, einen in seiner Dicke konstanten Lagerspalt zwischen der Lagerfläche 44 und dem Außenumfang 11 des Rotationskörpers 1 zu gewährleisten, wobei sich der konstante Lagerspalt wegen der allseitigen leichten Schwenkbarkeit des Lagerschuhs 42 automatisch einrichtet.

Auch die Axiallager 5, 5', 5" sind auf dieselbe Weise ausgestaltet wie die Radiallager 4, 4', sodaß auch die Axiallager 5, 5', 5" allseitig schwenkbar sind; lediglich die Lagerfläche 54 der jeweiligen Axiallager 5, 5', 5" ist nicht gekrümmt, sondern plan ausgebildet, um mit der planen Gegenlagerfläche 13 an der ersten Stirnseite 10 des Rotationskörpers 1 zusammenzuwirken. Auch hier sorgt die allseitige Schwenkbarkeit des jeweiligen Lagerschuhs 52 für eine automatische Einstellung einer konstanten Dicke des Lagerspalts.

Die axialen Ausgleichslager 6, 6', 6" sind ebenfalls mit einer entsprechenden allseitigen Schwenkbarkeit ausgestattet, wie dies in Verbindung mit den Radiallagern 4, 4' und den Axiallagern 5, 5', 5" beschrieben worden ist. Zusätzlich weisen die axialen Ausgleichslager 6, 6', 6", die mit ihrer jeweiligen Lagerfläche 64 mit einer an der zweiten Stirnseite 12 des Rotationskörpers 1 ausgebildete Gegenlagerfläche 15 zusammenwirken, jedoch noch eine Beweglichkeit zwischen der jeweiligen Lagerbasis 60 und dem jeweiligen Lagerschuh 62 in einer im unverschwenkten Zustand zu ihrer jeweiligen Lagerfläche senkrechten Richtung auf, sodaß bei diesen axialen Ausgleichslagern 6, 6', 6" nicht nur eine allseitige Verschwenkbarkeit gegeben ist, sondern außerdem der jeweilige Lagerschuh 62 zur Lagerbasis 60 hin oder von dieser weg bewegbar ist.

Der jeweilige Lagerschuh 62 des axialen Ausgleichslagers 6, 6', 6" ist mittels einer Federkraft, einer pneumatischen Kraft oder einer hydraulischen Kraft gegen die an der zweiten Stirnseite 12 des Rotationskörpers 1 ausgebildete Gegenlagerfläche derart vorgespannt, daß durch diese Vorspannung ein Ausgleich der vom jeweiligen aerostatischen Lagerdruck des als aerostatisches Lager ausgebildeten axialen Ausgleichslagers sowie des diesem gegenübergelegenen, ebenfalls als aerostatisches Lager ausgebildeten Axiallagers geschaffen ist. Dazu ist die Lagerbasis 60 zweiteilig ausgebildet, wobei ein erster Basisteil 60' mit dem stationären Körper 2 fest verbunden ist und wobei ein zweiter Basisteil 60" die konkave Fläche 61 aufweist, gegen die die konvexe Fläche 63 des Lagerschuhs 62 anliegt. Der den Lagerschuh 62 mit der Lagerbasis 60 verbindende Zuganker 65 ist als Kolben-Zylinder-Einheit ausgebildet, die zwar den Lagerschuh 62 und den zweitem Basisteil 60" in enger Anlage hält, die aber eine Bewegung zwischen der Einheit aus Lagerschuh 62 und zweiten Basisteil 60" bezüglich des ersten Lagerteils 60' in der zu den Lagerflächen 64, 15 senkrechten Richtung zuläßt. Außerdem ermöglicht der als Kolben-Zylinder-Einheit ausgebildete Zuganker 65 die Verschwenkung des Lagerschuhs 62 um den Kugelmittelpunkt, wie dies bereits in Verbindung mit dem Radiallager 4 beschrieben worden ist.

Das radiale Ausgleichslager 4" ist in der gleichen Weise aufgebaut wie die axialen Ausgleichslager 6, 6', 6", wobei aber der Lagerschuh des radialen Ausgleichslagers 4" bezüglich der Lagerbasis in Radialrichtung bewegbar ist. Dadurch stützt das schwenkbare und in Radialrichtung bewegbare radiale Ausgleichslager 4" zwar den Rotationskörper 1 gemeinsam mit den radialen Stützlagern 4 und 4' ab, ohne aber eine statische Überbestimmtheit der radialen Lagerung des Rotationskörpers 1 zu bewirken.

Sowohl die Axiallager 5, 5', 5", als auch die axialen Ausgleichslager 6, 6', 6" und die Radiallager 4, 4' sowie das radiale Ausgleichslager 4" sind in herkömmlicher Weise als aerostatische Lager ausgebildet, wobei diese aerostatischen Lager in der jeweiligen Lagerfläche 44, 54, 64 als Mikrolöcher ausgebildete Luftaustrittsdüsen 46 aufweisen. Diese Mikrolöcher sind in bekannter Weise durch hochenergetische Strahlung, beispielsweise mittels Laserstrahlung in die Lagerfläche eingebracht, wie dies beispielsweise aus der DE 44 36 15.6 C1 bekannt ist.

Fig. 4 zeigt in perspektivischer Ansicht beispielhaft einen Lagerschuh 42 mit dessen Lagerfläche 44. Anhand dieser Darstellung wird nachstehend eine besonders vorteilhafte Ausgestaltung eines aerostatischen Lagers dargestellt, bei welchem die Tragkraft über die Längserstreckung in Richtung des Pfeils Z in Fig. 4 zunimmt. Deutlich erkennbar ist in Fig. 4, daß die Luftaustrittsdüsen 46 für die Lagerluft einerseits entlang des Umfangs der Lagerfläche 44 entlang eines umfangsparallelen Rechtecks 46' angeordnet sind, daß andererseits aber auch quer zur Längsrichtung Z verlaufende Reihen 47, 48, 49, 50 von Austrittsdüsen für die Lagerluft vorgesehen sind. Der Abstand der jeweiligen Reihen 47, 48, 49, 50 zueinander verringert sich jeweils in Richtung des Pfeils Z, sodaß in dem in Fig. 4 auf der linken vorderen Seite der Lagerfläche 44 dargestellten Bereich mehr Luftaustrittsdüsen pro Flächeneinheit vorgesehen sind, als auf dem in Fig. 4 rechts hinten dargestellten Bereich. Hierdurch wird die Tragfähigkeit des Lagers 4 in dem Bereich erhöht, in welchem mehr Luftaustrittsdüsen pro Flächeneinheit vorgesehen sind. Das in Fig. 4 gezeigte Lager 4 weist daher eine in Richtung des Pfeils Z ansteigende Lager-Tragfähigkeit auf.

Bewegt sich nun eine in Fig. 4 nicht dargestellte Gegenlagerfläche mit hoher Geschwindigkeit in Richtung des Pfeils Z an der Lagerfläche 44 vorbei, so ist zwar der in Fig. 4 rechts hinten gezeigte Bereich der Lagerfläche 44 aufgrund der mit der Gegenlagerfläche bewegten oberflächennahen Luft bestrebt, von der Gegenlagerfläche abzuheben, weil die Luft in den Lagerspalt eindringt, doch bewirkt die höhere Tragfähigkeit im vorderen linken Bereich des Lagers 4, daß der hier aufgebaute höhere Lagerdruck aufgrund der um die Lagerung des Lagerschuhs 42 wirkenden Hebelkräfte, den hinteren, rückwärtigen Bereich der Lagerfläche 44 wieder zur Gegenlagerfläche hin bewegt, so daß der Lagerspalt über die Längserstreckung des Lagerschuhs 42 (in Richtung Z) konstant gehalten wird.

Auch wenn das Beispiel in Fig. 4 anhand eines Radiallagers 4 erläutert worden ist, sind die Lagerschuhe 52, 62 der Axiallager 5, 5', 5", der axialen Ausgleichslager 6, 6', 6" und des radialen Ausgleichslagers 4" mit der gleichen oder einer ähnlichen Anordnung der Luftaustrittsdüsen über die Längserstreckung der jeweiligen Lagerfläche ausgestaltet, sodaß auch dort die Tragfähigkeit des Lagers in Richtung der sich vorbeibewegenden Gegenlagerfläche erhöht ist.

Diese besondere Ausgestaltung des aerostatischen Lagers, wie es in Fig. 4 dargestellt ist, ist keinesfalls auf den Anwendungsfall einer Vorrichtung mit einem direkt angetriebenen Rotationskörper, beispielsweise einem Computertomographen, beschränkt; sie kann vielmehr überall dort vorgesehen werden, wo eine Relativbewegung zwischen dem Lagerschuh und der Gegenlagerfläche mit derart hoher Geschwindigkeit erfolgt, daß aerodynamische Effekte, die auf der hohen Geschwindigkeitsdifferenz zwischen den beiden gegenüberliegenden Lagerflächen beruhen, bestrebt sind, den Lagerschuh bezüglich der vorbeibewegten Gegenlagerfläche schräg zu stellen. Dabei muß der Lagerschuh nicht unbedingt allseitig schwenkbar gelagert sein; es kann bereits ausreichen, den Lagerschuh um eine Achse schwenkbar zu gestalten, die parallel zur Gegenlagerfläche und rechtwinklig zur Bewegungsrichtung des die Gegenlagerfläche aufweisenden bewegten Körpers verläuft, wie dies in Fig. 4 anhand der zylindrisch gekrümmten konvexen Fläche 43' an der Rückseite des Lagerschuhs 42 beispielhaft gezeigt ist.

Die Erfindung ist nicht auf das obige Ausführungsbeispiel beschränkt, das lediglich der allgemeinen Erläuterung des Kerngedankens der Erfindung dient. Im Rahmen des Schutzumfangs kann die erfindungsgemäße Vorrichtung vielmehr auch andere als die oben beschriebenen Ausgestaltungsformen annehmen. Die Vorrichtung kann hierbei insbesondere Merkmale aufweisen, die eine Kombination aus den jeweiligen Einzelmerkmalen der Ansprüche darstellen.

Bezugszeichen in den Ansprüchen, der Beschreibung und den Zeichnungen dienen lediglich dem besseren Verständnis der Erfindung und sollen den Schutzumfang nicht einschränken.

## Patentansprüche

1. Vorrichtung mit einem direkt angetriebenen Rotationskörper (1), der an seinem Umfang (11) bezüglich eines stationären Körpers (2) auf Radiallagern (4, 4') radial gelagert ist und der an zumindest einer ersten Stirnseite (10) bezüglich des stationären Körpers (2) axial gelagert ist, wobei zur axialen Lagerung aerostatische Lager (5, 5', 5") vorgesehen sind,
**dadurch gekennzeichnet**
- **dass** auf der ersten Stirnseite (10) eine zur Drehachse (X) des Rotationskörpers (1) koaxiale, ringförmige Magnetanordnung (32) ausgebildet ist und
- **dass** am stationären Körper (2), der ringförmigen Magnetanordnung (32) in Axialrichtung gegenüber gelegen, zumindest eine elektrische Spulenanordnung (30) vorgesehen ist, die zusammen mit der ringförmigen Magnetanordnung (32) Bestandteile eines elektrischen Direktantriebs (3) für den Rotationskörper (1) bildet.

2. Vorrichtung nach Anspruch 1,
**dadurch gekennzeichnet,**
**dass** der Rotationskörper (1) ringförmig gestaltet ist.

3. Vorrichtung nach Anspruch 1 oder 2,
**dadurch gekennzeichnet,**
**dass** auch die Radiallager (4, 4') durch aerostatische Lager gebildet sind.

4. Vorrichtung nach einem der vorhergehenden Ansprüche,
**dadurch gekennzeichnet,**
**dass** der von der ersten Stirnseite (10) des Rotationskörpers (1) abgewandten zweiten Stirnseite (12) zugeordnet zumindest ein aerostatisches axiales Ausgleichslager (6, 6', 6") vorgesehen ist, das jeweils zumindest einen in achsparalleler Richtung bewegbaren Lagerschuh (62) aufweist, der mittels einer Federkraft oder einer pneumatischen oder hydraulischen Kraft gegen die zweite Stirnseite (12) des Rotationskörpers (1) vorgespannt ist.

5. Vorrichtung nach einem der vorhergehenden Ansprüche,
**dadurch gekennzeichnet,**
**dass** unterhalb des Rotationskörpers (1) zumindest ein weiteres aerostatisches Radiallager (4") vorgesehen ist, das zumindest einen in radialer Richtung bewegbaren Lagerschuh aufweist, der mittels einer Federkraft oder einer pneumatischen oder hydraulischen Kraft gegen den Außenumfang (11) des Rotationskörpers (1) drückt.

6. Vorrichtung nach einem der vorhergehenden Ansprüche,
**dadurch gekennzeichnet,**
**dass** der ersten Stirnseite (10) des Rotationsantriebs (1) zugeordnet zumindest ein weiteres aerostatisches Axiallager vorgesehen ist, das zumindest einen in achsparalleler Richtung bewegbaren Lagerschuh aufweist, der mittels einer Federkraft oder einer pneumatischen oder hydraulischen Kraft gegen die erste Stirnseite (10) des Rotationskörpers (1) drückt.

7. Vorrichtung nach einem der vorhergehenden Ansprüche,
**dadurch gekennzeichnet,**
**dass** zumindest ein Teil der Axiallager (5, 5', 5", 6, 6', 6") jeweils zumindest einen schwenkbaren Lagerschuh (52, 62) aufweist, der um eine senkrecht zur Rotationsachse (X) des Rotationskörpers (1) verlaufende erste Achse schwenkbar ist.

8. Vorrichtung nach Anspruch 7,
**dadurch gekennzeichnet,**
**dass** der jeweilige schwenkbare Lagerschuh (52, 62) auch um eine zweite Achse schwenkbar ist, die senkrecht zu einer von der ersten Achse und der Rotationsachse (X) aufgespannten Ebene verläuft.

9. Vorrichtung nach einem der vorhergehenden Ansprüche,
**dadurch gekennzeichnet,**
**dass** zumindest ein Teil der Radiallager (4, 4', 4") jeweils zumindest einen schwenkbaren Lagerschuh (42) aufweist, der um eine parallel zur Rotationsachse (X) des Rotationskörpers (1) verlaufende erste Achse schwenkbar ist.

10. Vorrichtung nach Anspruch 9,
**dadurch gekennzeichnet,**
**dass** der jeweilige schwenkbare Lagerschuh (42) auch um eine zweite Achse schwenkbar ist, die parallel zu einer Tangente an den Umfang des Rotationskörpers (1) verläuft.

11. Vorrichtung nach einem der Ansprüche 7 bis 10,
**dadurch gekennzeichnet,**
**dass** die Lagerschuhe (42, 52, 62) so ausgestaltet sind, dass in Bewegungsrichtung der sich am Lagerschuh (42, 52, 62) vorbeibewegenden Gegenlagerfläche (14,13, 15) die Tragkraft des Lagers zunimmt.

12. Vorrichtung nach einem der Ansprüche 1 bis 11,
**dadurch gekennzeichnet,**
**dass** der stationäre Körper (2) von einer stationären Gantry einer bildgebenden Einrichtung, insbesondere eines Tomographen, gebildet ist und dass der Rotationskörper (1) von einer rotierenden Gantry der bildgebenden Einrichtung gebildet ist.

13. Vorrichtung nach einem der Ansprüche 4 bis 12 mit einer relativ zu dem zumindest einen Lagerschuh (42, 52, 62) in einer hauptrichtung bewegten begenlagerfläche (14, 13,15),
**dadurch gekennzeichnet, dass** der zumindest eine Lagerschuh (42, 52,62) eine Lagerfläche (44) mit daraustrittsöfnungen (46) aufweist,
dass der zumindest eine Lagerschuh (42, 52, 62) um eine erste Achse, die rechtwinklig zur Hauptbewegungsrichtung (Z) der Gegenlagerfläche und parallel zur Gegenlagerfläche verläuft, schwenkbar ist,
dass der Lagerschuh (42, 52, 62) so ausgestaltet ist, dass in Bewegungsrichtung der sich am Lagerschuh (42, 52, 62) vorbeibewegenden Gegenlagerfläche die Tragkraft des Lagers zunimmt,
wobei der Lagerschuh (42, 52, 62) vorzugsweise auch um eine zweite Achse schwenkbar ist, die rechtwinklig zur ersten Achse und parallel zur Gegenlagerfläche verläuft.

14. Vorrichtung nach einem der vorhergehenden Ansprüche,
**dadurch gekennzeichnet,**
**dass** der schwenkbare Lagerschuh (42, 52, 62) ein Kugelkopflager aufweist und so beliebig schwenkbar ist.

15. Vorrichtung nach einem der Ansprüche 13 oder 14,
**dadurch gekennzeichnet,**
**dass** die Anzahl der Gasaustrittsöffnungen (46) in der Lagerfläche (44) des Lagerschuhs (42) in Bewegungsrichtung der sich am Lagerschuh (42) vorbeibewegenden Gegenlagerfläche zunimmt.

## Claims

1. Device with a directly driven rotary body (1), which at its periphery (11) is mounted radially relative to a stationary body (2) on radial bearings (4, 4') and which at at least one first face (10) is mounted axially relative to the stationary body (2), wherein aerostatic bearings (5, 5', 5") are provided for the axial mounting,
**characterised in that**
• an annular magnet arrangement (32) coaxial to the rotational axis (X) of the rotary body (1) is configured on the first face (10) and
• that located opposite the annular magnet arrangement (32) in axial direction on the stationary body (2), there is provided at least one electric coil arrangement (30), which together with the annular magnet arrangement (32) forms components of an electric direct drive (3) for the rotary body (1).

2. Device according to claim 1, **characterised in that** the rotary body (1) is annular in configuration.

3. Device according to claim 1 or 2, **characterised in that** the radial bearings (4, 4') are also formed by aerostatic bearings.

4. Device according to one of the preceding claims, **characterised in that** at least one aerostatic axial compensating bearing (6, 6', 6") associated with the second face (12) directed away from the first face (10) of the rotary body (1) is provided, which respectively has at least one bearing shoe (62) movable in axis-parallel direction, which is biased against the second face (12) of the rotary body (1) by means of a spring force or a pneumatic or hydraulic force.

5. Device according to one of the preceding claims, **characterised in that** provided below the rotary body (1) is at least one further aerostatic radial bearing (4"), which has at least one bearing shoe movable in radial direction, which presses against the outer periphery (11) of the rotary body (1) by means of a spring force or a pneumatic or hydraulic force.

6. Device according to one of the preceding claims, **characterised in that** at least one further aerostatic axial bearing associated with the first face (10) of the rotary drive (1) is provided, which has at least one bearing shoe movable in axis-parallel direction, which presses against the first face (10) of the rotary body (1) by means of a spring force or a pneumatic or hydraulic force.

7. Device according to one of the preceding claims, **characterised in that** at least a portion of the axial bearings (5, 5', 5", 6, 6' 6") respectively have at least one pivoting bearing shoe (52, 62), which is pivotable about a first axis running perpendicularly to the rotational axis (X) of the rotary body (1).

8. Device according to claim 7, **characterised in that** the respective pivoting bearing shoe (52, 62) is pivotable about a second axis, which runs perpendicularly to a plane spanned by the first axis and the rotational axis (X).

9. Device according to one of the preceding claims, **characterised in that** at least a portion of the radial bearings (4, 4', 4") respectively have at least one pivoting bearing shoe (42), which is pivotable about a first axis running parallel to the rotational axis (X) of the rotary body (1).

10. Device according to claim 9, **characterised in that** the respective pivoting bearing shoe (42) is also able to pivot around a second axis, which runs parallel to a tangent to the periphery of the rotary body (1).

11. Device according to one of claims 7 to 10, **characterised in that** the bearing shoes (42, 52, 62) are configured such that the loading capacity of the bearing increases in the direction of movement of the counter-bearing surface (14, 13, 15) moving past the bearing shoe (42, 52, 62).

12. Device according to one of claims 1 to 11, **characterised in that** the stationary body (2) is formed by a stationary gantry of an imaging device, in particular a tomograph, and that the rotary body (1) is formed by a rotating gantry of the imaging device.

13. Device according to one of claims 4 to 12 with a counter-bearing surface (14, 13, 15) moved relative to the at least one bearing shoe (42, 52, 62) in a main direction,
**characterised in that** the at least one bearing shoe (42, 52, 62) has a bearing surface (44) with gas outlets (46),
that the at least one bearing shoe (42, 52, 62) is pivotable about a first axis, which runs at right angles to the main direction of movement (Z) of the counter-bearing surface and parallel to the counter-bearing surface,
that the bearing shoe (42, 52, 62) is configured such that the loading capacity of the bearing increases in the direction of movement of the counter-bearing surface moving past the bearing shoe (42, 52, 62),
wherein the bearing shoe (42, 52, 62) is preferably also pivotable about a second axis, which runs at right angles to the first axis and parallel to the counter-bearing surface.

14. Device according to one of the preceding claims, **characterised in that** the pivotable bearing shoe (42, 52, 62) has a ball head bearing and thus can be pivoted as desired.

15. Device according to one of claims 13 or 14, **characterised in that** the number of gas outlets (46) in the bearing surface (44) of the bearing shoe (42) increases in the direction of movement of the counter-bearing surface moving past the bearing shoe (42).

## Revendications

1. Dispositif comprenant un corps rotatif (1) à entraînement direct, qui est monté à sa périphérie (11) radialement sur des paliers radiaux (4, 4') par rapport à un corps stationnaire (2) et qui est monté, au moins à une première face frontale (10) axialement par rapport au corps stationnaire (2), dans lequel il est prévu des paliers aérostatiques (5, 5', 5") pour le montage axial,
**caractérisé en ce que**
- un agencement magnétique (32) de forme annulaire et coaxial à l'axe de rotation (X) du corps rotatif (1) est réalisé sur la première face frontale (10), et
- il est prévu sur le corps stationnaire (2) et en disposition opposée à l'agencement magnétique annulaire (32) en direction axiale, au moins un agencement à bobine électrique (30) qui, conjointement avec l'agencement magnétique annulaire (32), forment les éléments constitutifs d'un entraînement direct électrique (3) pour le corps rotatif (1).

2. Dispositif selon la revendication 1,
**caractérisé en ce que** le corps rotatif (1) est conçu sous forme annulaire.

3. Dispositif selon la revendication 1 ou 2,
**caractérisé en ce que** les paliers radiaux (4, 4') sont également formés par des paliers aérostatiques.

4. Dispositif selon l'une des revendications précédentes,
**caractérisé en ce qu'**il est prévu au moins un palier de compensation axial aérostatique (6, 6', 6") associé à la seconde face frontale (12) détournée de la première face frontale (10) du corps rotatif (1), ledit palier comprenant au moins un sabot de palier (62) déplaçable en direction parallèle à l'axe, qui est précontraint au moyen d'une force élastique ou d'une force pneumatique ou hydraulique contre la seconde face frontale (12) du corps rotatif (1).

5. Dispositif selon l'une des revendications précédentes,
**caractérisé en ce que**, au-dessous du corps rotatif (1), il est prévu au moins un autre palier radial aérostatique (4"), qui comprend au moins un sabot de palier déplaçable en direction radiale, qui pousse contre la périphérie extérieure (11) du corps rotatif (1) au moyen d'une force élastique ou d'une force pneumatique ou hydraulique.

6. Dispositif selon l'une des revendications précédentes,
**caractérisé en ce qu'**il est prévu au moins un autre palier axial aérostatique, associé à la première face frontale (10) du corps rotatif (1), ledit palier comprenant au moins un sabot de palier déplaçable en direction parallèle à l'axe, qui pousse contre la première face frontale (10) du corps rotatif (1) au moyen d'une force élastique ou d'une force pneumatique ou hydraulique.

7. Dispositif selon l'une des revendications précédentes,
**caractérisé en ce qu'**une partie au moins des paliers axiaux (5, 5', 5", 6, 6', 6") comprennent chacun au moins un sabot de palier pivotant (52, 62), qui est capable de pivoter autour d'un premier axe qui s'étend perpendiculairement à l'axe de rotation (X) du corps rotatif (1).

8. Dispositif selon la revendication 7,
**caractérisé en ce que** le sabot de palier pivotant respectif (52, 62) est également capable de pivoter autour d'un second axe qui s'étend perpendiculairement à un plan défini par le premier axe et par l'axe de rotation (X).

9. Dispositif selon l'une des revendications précédentes,
**caractérisé en ce qu'**une partie au moins des paliers radiaux (4, 4', 4") comprennent chacun au moins un sabot de palier pivotant (42), qui est capable de pivoter autour d'un premier axe qui s'étend parallèlement à l'axe de rotation (X) du corps rotatif (1).

10. Dispositif selon la revendication 9,
**caractérisé en ce que** le sabot de palier pivotant respectif (42) est également capable de pivoter autour d'un second axe qui s'étend parallèlement à une tangente à la périphérie du corps rotatif (1).

11. Dispositif selon l'une des revendications 7 à 10,
**caractérisé en ce que** les sabots de paliers (42, 52, 62) sont ainsi conçus que la force portante du palier augmente dans la direction de déplacement de la surface de palier antagoniste (14, 13, 15) qui se déplace devant le sabot de palier (42, 52, 62).

12. Dispositif selon l'une des revendications 1 à 11,
**caractérisé en ce que** le corps stationnaire (2) est formé par un portique stationnaire d'un système d'imagerie, en particulier d'un tomographe, et **en ce que** le corps rotatif (1) est formé par un portique rotatif du système d'imagerie.

13. Dispositif selon l'une des revendications 4 à 12, comprenant une surface de palier antagoniste (14, 13, 15) déplacée dans une direction principale par rapport audit au moins un sabot de palier (42, 52, 62),
**caractérisé en ce que** ledit au moins un sabot de palier (42, 52, 62) comprend une surface de palier (44) avec des ouvertures de sortie de gaz (46),
**en ce que** ledit au moins un sabot de palier (42, 52, 62) est capable de pivoter autour d'un premier axe, qui s'étend perpendiculairement à la direction de déplacement principale (Z) de la surface de palier antagoniste et parallèlement à la surface de palier antagoniste,
**en ce que** le sabot de palier (42, 52, 62) est ainsi conçu que la force portante du palier augmente dans la direction de déplacement de la surface de palier antagoniste qui se déplace devant le sabot de palier (42, 52, 62),
dans lequel le sabot de palier (42, 52, 62) est de préférence également capable de pivoter autour d'un second axe qui s'étend perpendiculairement au premier axe et parallèlement à la surface de palier antagoniste.

14. Dispositif selon l'une des revendications précédentes,
**caractérisé en ce que** le sabot de palier pivotant (42, 52, 62) comprend un palier à tête sphérique et est ainsi capable de pivoter dans tous les sens.

15. Dispositif selon l'une des revendications 13 ou 14,
**caractérisé en ce que** le nombre des ouvertures de sortie de gaz (46) dans la surface de palier (44) du sabot de palier (42) augmente dans la direction de déplacement de la surface de palier antagoniste qui se déplace devant le sabot de palier (42).
